Europäisches Patentamt

(19) European Patent Office

Office européen des brevets

(11) EP 1 287 158 B1

(12) EUROPEAN PATENT SPECIFICATION

(45) Date of publication and mention
of the grant of the patent:
11.08.2004 Bulletin 2004/33

(51) Int Cl.⁷: **C12Q 1/18**, C12N 15/00

(86) International application number:
PCT/GB2001/002400

(21) Application number: 01934165.0

(22) Date of filing: 30.05.2001

(87) International publication number:
WO 2001/092559 (06.12.2001 Gazette 2001/49)

(54) **SCREENING SYSTEM FOR ANTIBIOTICS**

SCREENINGSYSTEM FÜR ANTIBIOTIKA

SYSTEME DE CRIBLAGE POUR ANTIBIOTIQUES

(84) Designated Contracting States:
**AT BE CH CY DE DK ES FI FR GB GR IE IT LI LU
MC NL PT SE TR**
Designated Extension States:
**AL LT LV MK RO SI**

(30) Priority: 01.06.2000 GB 0013384

(43) Date of publication of application:
05.03.2003 Bulletin 2003/10

(73) Proprietor: **Plant Bioscience Limited
Norwich, Norfolk NR4 7UH (GB)**

(72) Inventors:
• **HONG, Hee-Jeon
Norfolk NR2 3EW (GB)**
• **PAGET, Mark, Sebastian, Boye
Norfolk NR2 3LD (GB)**
• **BUTTNER, Mark, Jeremy
Norwich, Norfolk NR2 2BB (GB)**

(74) Representative: **Kremer, Simon Mark et al
Mewburn Ellis LLP
York House,
23 Kingsway
London WC2B 6HP (GB)**

(56) References cited:
**WO-A-97/23504**

• **PAGET MARK S B ET AL: "A putative
two-component signal transduction system
regulates sigmaE, a sigma factor required for
normal cell wall integrity in Streptomyces
coelicolor A3(2)." MOLECULAR
MICROBIOLOGY, vol. 33, no. 1, July 1999
(1999-07), pages 97-107, XP001074620 ISSN:
0950-382X cited in the application**
• **PAGET MARK S B ET AL: "Evidence that the
extracytoplasmic function sigma factor sigmaE
is required for normal cell wall structure in
Streptomyces coelicolor A3(2)." JOURNAL OF
BACTERIOLOGY, vol. 181, no. 1, January 1999
(1999-01), pages 204-211, XP001074661 ISSN:
0021-9193 cited in the application**
• **ULIJASZ ANDREW T ET AL: "A
vancomycin-inducible lacZ reporter system in
Bacillus subtilis: Induction by antibiotics that
inhibit cell wall synthesis and by lysozyme."
JOURNAL OF BACTERIOLOGY, vol. 178, no. 21,
1996, pages 6305-6309, XP001074640 ISSN:
0021-9193 cited in the application**
• **SUGIURA AKEMI ET AL: "Signal-sensing
mechanisms of the putative osmosensor KdpD
in Escherichia coli." MOLECULAR
MICROBIOLOGY, vol. 14, no. 5, 1994, pages
929-938, XP008002522 ISSN: 0950-382X cited in
the application**
• **WALDERHAUG M O ET AL: "KDPD AND KDPE,
PROTEINS THAT CONTROL EXPRESSION OF
THE KDPABC OPERON, ARE MEMBERS OF THE
TWO-COMPONENT SENSOR-EFFECTOR
CLASS OF REGULATORS" JOURNAL OF
BACTERIOLOGY, WASHINGTON, DC, US, vol.
174, no. 7, 1 April 1992 (1992-04-01), pages
2152-2159, XP000197284 ISSN: 0021-9193 cited
in the application**

**Description**

Technical Field

The present invention relates to methods and materials for screening for compounds which have potential as antibiotics. It further relates to methods for generating microorganisms having utility in screening, tools which can be generally used in such methods, the microorganisms themselves, and biosensing methods employing the microorganisms.

Background Art

There is an ongoing requirement for novel compounds that have antibiotic activity, for instance to counteract the problem of drug resistance. Methods for screening potential sources of antibiotic that have been used in the prior art include those which are based on particular 'indicator' or 'reporter' strains of bacteria.

For instance, the use of *E. coli* PG8 (lacking both chromosomal β-lactamase and the cell wall biosynthetic enzyme PBP 1B) as an indicator strain led to the discovery of the monocyclic β-lactams [the nocardicins (Aoki *et al.,* 1976) and the monobactams (Imada *et al.,* 1981)].

Another screening approach has exploited the fact that the β-lactamase of *Bacillus licheniformis* is induced by β-lactams; the β-lactamase produced is easily detected by a chromogenic reaction, and this reporter system led to the independent discovery of the monobactams (Sykes *et al.*, 1981).

Ulijasz et al. (1996) disclose a two component signal transduction system that is based on VanS/VanR which activates transcription from the vanH promoter (vanHp) in *Enterococcus faecium*.

It can thus be seen that novel indicator systems would provide a contribution to the art.

Disclosure of the invention

The present inventors have provided a novel system to provide a broad-range, generic screen for antibiotics, lytic enzymes and other compounds which target the cell envelope.

The system is based on the use of a promoter, linked to a suitable reporter gene, which promoter is regulated by a two-component signal transduction system. The two components are (i) a cell membrane located sensor, and (ii) a trans-acting factor which is activated in response to a stimulus to the sensor such that it induces transcription of the reporter gene linked to the promoter.

As a test system, the inventors have established that the promoter from the gene encoding $\sigma^E$ (*sigE*) can be used as a generic detector of antibiotics or enzymes that interfere with the physical integrity of the cell envelope when tested in *Streptomyces coelicolor A3(2).*

It was already known that the gene encoding *sigE* was regulated at the level of transcription by a two-component signal transduction system (CseB and CseC) (Paget *et al.*, 1999b). However, although the *sigE* transcription factor ($\sigma^E$) was believed to control certain cell wall-related functions in *S. coelicolor* A3(2) in response to changes in the cell envelope (Lonetto *et al.,* 1994; Paget *et al.*, 1999a; Paget *et al.*, 1999b) the precise nature of the initiating signal was unknown. There was no suggestion in any of these publications that the system had utility as a screening agent.

Thus in a first aspect of the invention there is disclosed a method of detecting an activity of an antibiotic analyte in a sample comprising the steps of:

(a) providing a transformed microorganism which comprises a nucleic acid encoding a promoter which is regulated by a two-component signal transduction system operably linked to a heterologous reporter gene capable of causing a detectable signal,
(b) contacting the sample with the transformed microorganism,
(c) observing said bacterium for said detectable signal;

The term "antibiotic" is used broadly in this aspect to cover all antimicrobial compounds (natural, semi-synthetic or synthetic) which inhibit or kill (susceptible) microorganisms and to embrace compounds which interfere with the physical integrity of the cell envelope. Suitable antibiotics include but are not limited to penicillins (such as penicillin G, amoxycillin and ticarcillin), glycopeptides (such as teicoplanin, ristocetin and vancomycin) and peptides such as bacitracin. Glycopeptides and peptide antibiotics are preferred. As stated above, the method can also be used to detect activity of lytic enzymes. Where the term "antibiotic" is used hereinafter, the skilled person will appreciate that this applies mutatis mutandis to such enzymes.

The activity detected may be correlated with the presence or absence of an antibiotic, or putative antibiotic, in the sample in a qualitative manner. Alternatively it may be used to make a quantitative assessment.

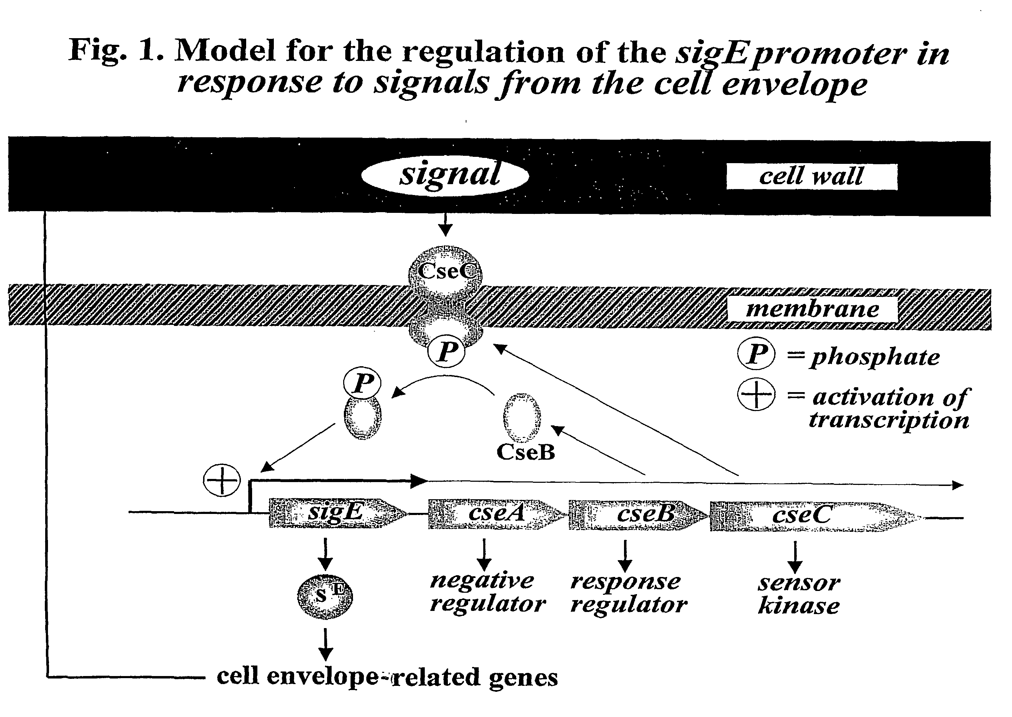

As described above, the "two components" are (i) a sensor (e.g. receptor), which will be receptive to changes in the cell envelope or membrane of the microorganism (and will generally be membrane bound) and (ii) a trans-acting factor which is activated (e.g. phosphorylated) in response to stimulation of the sensor such that it activates the promoter and induces transcription of the reporter gene linked to the promoter. An example of such a system is shown in Fig 1, which shows the *sigE* promoter, which is regulated by CseB and CseC.

The term "operably linked" refers to the linkage of a promoter to an RNA-encoding DNA sequence, and especially to the ability of the promoter to induce production of RNA transcripts corresponding to the DNA sequence when the promoter or regulatory sequence is recognized by a suitable polymerase. The term means that linked DNA sequences (e.g. promoter, reporter gene, terminator sequence) are operational or functional, i.e. work for their intended purposes.

By "observing" is meant ascertaining by any means (directly or indirectly) the presence or absence of the selected signal which is indicative of the binding event.

Thus the invention provides, *inter alia,* a method of detecting in a sample the activity of an antibiotic which affects cell integrity, which method comprises the steps of:(a) providing a transformed microorganism which comprises a nucleic acid encoding a promoter operably linked to a heterologous reporter gene capable of causing a detectable signal,(b) contacting the sample with the transformed microorganism, (c) observing said microorganism for said detectable signal, wherein the promoter is regulated by a two-component signal transduction system, which signal components are (i) a receptor responsive to changes in the cell envelope or membrane of the microorganism (ii) a trans-acting factor which is activated in response to stimulation by the receptor, and which regulates the promoter.

Some particular embodiments and aspects will now be discussed in more detail.

*Choice of sample*

Samples may be selected from any suitable source. In particular, samples may be selected from culture supernatants and extracts from soil isolates, compounds produced by chemical synthesis including combinatorial chemistry; and compounds produced by combinatorial biosynthesis.

By isolating the antibiotic from the sample of the above method, an isolated antibiotic which affects cell integrity can be produced.

*Choice of promoter*

In one embodiment, the promoter is the *sigE* promoter or an active variant thereof. The nucleotide sequence of the *sigE* operon is published (Paget *et al.*, 1999b).

'Variants' in this context will have promoter activity, by which is meant the ability to bind an RNA polymerase (and other factors that initiate or modulate transcription under the e.g. *sigE* promoter) whereby an RNA transcript is produced from the reporter gene under the appropriate conditions i.e. activation of the component signal transduction pathway.

Variants may be modified from an 'authentic' native sequence e.g. by introducing changes into the full-length or part-length sequence, for example substitutions, insertions, and/or deletions. This may be achieved by any appropriate technique, including restriction of the sequence with an endonuclease followed by the insertion of a selected base sequence (using linkers if required) and ligation. Also possible is PCR-mediated mutagenesis using mutant primers.

It may, for instance, be preferable to add in or remove restriction sites in order to facilitate further cloning. Modified sequences according to the present invention may have a sequence at least 70% identical to the sequence of the full or part-length inducible promoter or operon protein as appropriate. Typically there is 80% or more, 90% or more 95% or more or 98% or more identity between the modified sequence and the authentic sequence. There may be up to five, for example up to ten or up to twenty or more nucleotide deletions, insertions and/or substitutions made to the full-length or part length sequence provided functionality is not totally lost.

Similarity or identity may be as defined and determined by the TBLASTN program, of Altschul *et al.* (1990) *J. Mol. Biol.* 215: 403-10, or BestFit, which is part of the Wisconsin Package, Version 8, September 1994, (Genetics Computer Group, 575 Science Drive, Madison, Wisconsin, USA, Wisconsin 53711). Preferably sequence comparisons are made using FASTA and FASTP (see Pearson & Lipman, 1988. Methods in Enzymology 183: 63-98). Parameters are preferably set, using the default matrix, as follows:

Gapopen (penalty for the first residue in a gap): -16 for DNA Gapext (penalty for additional residues in a gap): -4 for DNA KTUP word length: 6 for DNA.

Alternatively, homology in this context can be judged by probing under appropriate stringency conditions. One common formula for calculating the stringency conditions required to achieve hybridization between nucleic acid molecules of a specified sequence homology is (Sambrook et al., 1989):

$$T_m = 81.5°C + 16.6\text{Log} [Na+] + 0.41 (\% \text{ G+C}) - 0.63 (\% \text{ formamide})$$

$$600/\#bp \text{ in duplex.}$$

As an illustration of the above formula, using [Na+] = [0.368] and 50-% formamide, with GC content of 42% and an average probe size of 200 bases, the $T_m$ is 57°C. The $T_m$ of a DNA duplex decreases by 1 - 1.5°C with every 1% decrease in homology. Thus, targets with greater than about 75% sequence identity would be observed using a hybridization temperature of 42°C. Such a sequence would be considered substantially homologous to the nucleic acid sequence of the present invention.

Use of the promoters discussed above, particularly the *sigE* promoter or an active variant thereof, in the methods or systems described herein, forms one aspect of the present invention.

*Choice of reporter gene*

Generally the observed signal arises in consequence to an increased expression of a reporter protein from the reporter gene.

In the examples herein, the reporter protein provides resistance against a particular antibiotic (or otherwise lethal concentration of antibiotic). The detection in this case is the viability and/or increased rate of growth of the host microorganism in the presence of the antibiotic. This can be visualised e.g. directly, as a bacterial lawn on a nutrient plate. An example is the neo gene which confers resistance to both neomycin and kanamycin.

Alternative reporter genes may be used for increased ease of scoring and/or sensitivity. Most preferably the activity of the signal protein, or the protein itself, can be estimated photometrically (especially by fluorimetry or luminometry).

This may be directly e.g. using instance green (and red) fluorescent protein, insect luciferase, and photobacterial luciferase. Alternatively it may be indirect e.g. whereby the signal gene causes a change which is detected by a colour indicator e.g. a pH change. In particular, the *lux* genes of *Vibrio harveyi* have been used successfully in *Streptomyces.*

Other suitable signal proteins (which have a readily detectable activity) are known in the art e.g. β-galactosidase, which can generate a coloured substrate. The signal may utilise cofactors.

Methods for introducing signal genes into appropriate hosts are described in further detail below.

*Integration of reporter gene*

In one embodiment, the reporter gene may be introduced into the host such that it is operably linked to an appropriate existing inducible promoter. Typically this will be achieved by initiating targeted integration using aspects of the sequence forming part of the promoter region or operon.

Direct integration of a signal gene system such as luciferase (e.g. luxAB operon) into an environmentally responsive regulon in the host microorganism can be achieved by transposition or by illegitimate or legitimate recombination between a genetic construct introduced into the cell and the target operon or gene cluster located on either the chromosome or an episomal element.

In such cases, although the promoter is native to the cell, the reporter gene is "heterologous" to the promoter, by which is meant that the gene in question has been introduced into the cell using genetic engineering, i.e. by human intervention. Generally the heterologous gene will be non-naturally occurring in cells of that type.

*Nucleic acid constructs*

Preferably the nucleic acid encoding the promoter operably linked to the heterologous reporter gene capable of causing a detectable signal is in the form of an extrachromosomal vector.

"Vector", unless further specified, is defined to include, inter alia, any plasmid DNA, lysogenic phage DNA and/or transposon DNA, in double or single stranded linear or circular form which may or may not be self transmissible or mobilizable, and which can transform a prokaryotic or eukaryotic host either by integration into the cellular genome or exist extrachromosomally (e.g. autonomous replicating plasmid with an origin of replication).

Strain derivatives encoding different gene dosage levels of the promoter/signal gene can be created by integration of the construct into the chromosome (low copy number/low sensitivity) or by use of medium or high copy number plasmids (medium or high sensitivity).

One example herein is based on the neo reporter plasmid, pIJ486, which was published in 1986 (Ward *et al.,*

1986) and has been used widely in the *Streptomyces* community to identify promoter-containing fragments and to quantify the strength of promoters *in vivo*. It has also been used to identify compounds which induce given promoters (e.g. Salah-Bey *et al.*, 1995; Murakami *et al.*, 1989). This has been used to generate the *sigEp-neo* fusion plasmid (pIJ6880) as described hereinafter.

Generally speaking, those skilled in the art are well able to construct vectors and design protocols for recombinant gene expression in common bacterial hosts. Suitable vectors can be chosen or constructed, containing appropriate regulatory sequences, including promoter sequences, terminator fragments, polyadenylation sequences, enhancer sequences and other sequences as appropriate. For further details see, for example, *Molecular Cloning: a Laboratory Manual:* 2nd edition, Sambrook *et al*, 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in *Current Protocols in Molecular Biology,* Second Edition, Ausubel et al. eds., John Wiley & Sons, 1992.

Vectors for use in the invention will typically include: (a) a promoter which is regulated by a two-component signal transduction system operably linked to, (b) a heterologous reporter gene capable of causing a detectable signal, wherein the "two components" are (i) a sensor, which will be receptive to changes in the cell envelope or membrane of the microorganism and (ii) a trans-acting factor which is activated in response to stimulation of the sensor such that it activates the promoter.

In one assay described hereinafter, the *sigE* promoter is on a plasmid replicon with a copy number of 100-200 per chromosome. However the gene (*cseB*) encoding the activator of the promoter is present in single copy in the chromosome. In order to improve sensitivity it may be desired to increase the copy number of all or some of the genes encoding the two component system, which will generally be part of the promoter's native operon. For instance, in the assay employed in the examples below, additional copies of *cseB* may enhance the sensitivity of the assay. Similarly, changing the copy number of the sensor kinase gene (*cseC*) or of *cseA* (which is believed to function as a negative regulator of the system) might have this effect.

One way of increasing copy number would be for the operon genes(optionally modified) to be included in a vector, optionally operably linked to a strong promoter or even the inducible promoter. The vector may or may not be the same vector as that carrying the inducible promoter and reporter gene.

Vectors as described above form one aspect of the present invention, as do methods of using them to produce a transformed host cell. The vector may remain discrete in the host. Alternatively it may integrate into the genome of the host.

*Choice of host strain*

The assay may use any suitable species of bacteria. Preferably the assay uses a strain of *Streptomyces* e. g. M600, which is a plasmid-free derivative of wild-type *S. coelicolor* A3(2). The *sigEp-neo* fusion plasmid (pIJ6880) may then be introduced into the strain in question in order to perform the invention.

However it may be preferred to use other *Streptomyces* species that may have greater sensitivity to cell wall-specific antibiotics. For instance, specialised *S. coelicolor* host mutants may be employed that are defective in the cell wall and therefore hypersensitive to cell wall-specific antibiotics.

In addition, it may be preferred to use strains in which enzymes which may otherwise degrade antibiotics (thereby reducing the sensitivity of the assay) have been inactivated.

For example, it is known that *S. coelicolor* produces one or more β-lactamases (enzymes that inactivate β-lactams), and construction of a specialised host in which the β-lactamase structural genes have been inactivated would increase sensitivity of the bioassay in detecting β-lactams. The availability of the *S. coelicolor* genome sequence (at the 'sanger.ac.uk/Projects/S_coelicolor' website) means that the identification of host genes for disruption is straightforward; for example, there are putative β-lactamase structural genes on cosmids H63 and J11. Similarly, there is a putative vancomycin resistance locus on cosmid 66T3, and disruption of this locus might increase sensitivity of the bioassay in detecting vancomycin-related glycopeptides.

It may also be preferred to use genera of bacteria in which the promoter employed in the invention is non-native. This may permit different spectra of inducing compounds to be revealed. In such cases it may be required to express other heterologous genes in the bacteria in order to ensure the functionality of the two-component assay system. Generally the other genes may include transport and binding proteins, as well as the sensor and trans-acting factor(s).

As described above, the operon could be present on the same vector as that carrying the promoter and reporter gene, if preferred.

Examples of suitable bacteria may include, for example, the mycobacteria which are closely related to the streptomycetes, and include important human pathogens such as *Mycobacterium tuberculosis* and *Mycobacterium leprae.* The four-gene *sigE* operon (in single copy) and the *sigEp-neo* fusion (in multicopy) could be expressed in *Mycobacterium smegmatis* to permit screening therein.

Preferably, however, the host microorganism is the same species as that which provided the source of the inducible promoter, and will therefore naturally express the other components of the system required to give screening function.

The host cells described above, which may include vectors of the present invention, or heterologous reporter genes, form a further aspect of the present invention. An example transformed microorganism for use in a method described above would be one which is transformed with a vector comprising (a) a promoter operably linked to (b) a heterologous reporter gene capable of causing a detectable signal, wherein the promoter is regulated by a two-component signal transduction system, which signal components are (i) a receptor responsive to changes in the cell envelope or membrane of the microorganism (ii) a trans-acting factor which is activated in response to stimulation by the receptor, and which regulates the promoter.

*Kits*

Also embraced within the scope of the present invention are kits for performing the various aspects of the invention. For instance a kit suitable for use in the first aspect may comprise a preparation of the microorganism, plus further means for carrying out the contact or observation steps e.g. buffers, cofactors (e.g. luciferin for addition to luciferase).

Thus in a further aspect of the present invention there is provided a kit for detecting an activity of an antibiotic in a sample comprising:

    (a) a transformed microorganism as described above,
    (b) means for detecting the signal produced from the reporter gene.

For example, where the signal is bacterial luciferase, this may be detected extracellularly using a photomultiplier or photodiode or any other photosensitive device.

Where the reporter gene encodes an antibiotic resistance gene (e.g. the neo gene in the Examples below) this could be used in parallel processing system in which assays for multiple potential inducers are assessed using multiple nutrient plates. Alternatively, using an automated 96-well microtitre dish assay, kanamycin-resistant growth may be assayed automatically by optical density.

Naturally the methods and kits of the invention described above could be used as a primary screen, with further screens (e.g. based on antibiosis of target organisms, which may be different species to the screening microorganism) being employed to further exclude compounds not having the desired activity.

The invention will now be further described with reference to the following Figures and Examples.

Figures and Tables

Figure 1 shows a model for regulation of the *sigE* promoter in response to signals from the cell envelope.

Figure 2 shows induction of the *sigE* promoter by cell wall-specific antibodies.

Table 1 summarises the results of the experiment described in Example 2, in which 20 antibiotics were tested for ability to induce the *sigE* promoter.

Examples

Example 1 - construction of reporter construct

A ~750bp *Pvu*II-*Sma*I fragment carrying the *sigE* promoter (*sigEp*), was blunt-end cloned into pIJ2925 (Janssen and Bibb, 1993) cut with *Sma*I and *Eco*RI to create pIJ5953.

The *sigEp* fragment was re-isolated from pIJ5953 as a ~750bp *Bgl*II fragment and cloned into the multicopy promoter probe plasmid pIJ486 (Ward *et al.*, 1986) cut with *Bam*HI, such that expression of the vector aminoglycoside phosphotransferase gene (*neo*), which confers resistance to both neomycin and kanamycin, depends on *sigEp.* The orientation of the insert was determined by digestion with *Sph*I. The resulting plasmid was designated pIJ6880.

<u>Example 2 - construction and use testing of reporter strain</u>

Plasmid pIJ6880 was introduced by protoplast transformation (Hopwood et al., 1985) into M600 (Chakraburtty and Bibb, 1997), a plasmid-free derivative of wild-type *S. coelicolor* A3(2), and was found to confer resistance to approximately 80 $\mu$g ml$^{-1}$ kanamycin (on MMT medium; Katz *et al.*, 1983).

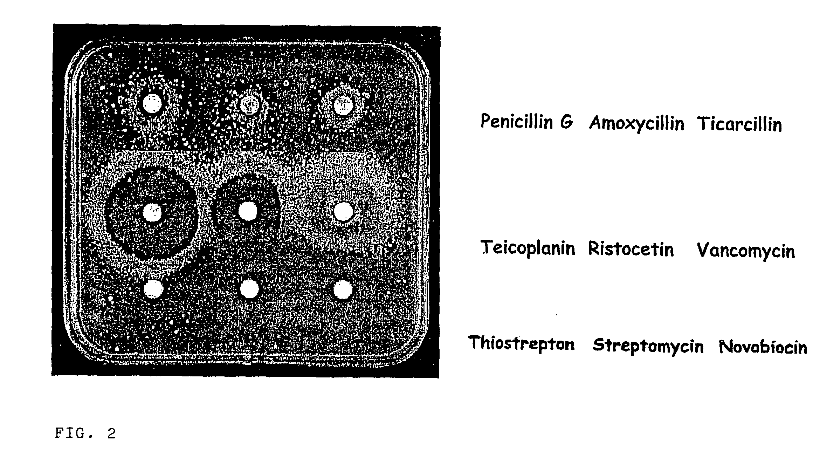

To see if the *sigE* promoter could be induced by control-antibiotics known to target the cell envelope, spores of M600 carrying pIJ6880 were spread on MMT medium carrying a lethal concentration of kanamycin (100 $\mu$g ml$^{-1}$) and potential inducers were applied on paper discs to the freshly spread plates. The results are shown for nine antibiotics in Figure 2. Inducers of the *sigE* promoter (penicillin G, amoxycillin, ticarcillin, teicoplanin, ristocetin and vancomycin) raised the level of expression of the *neo* gene and hence induced a halo of kanamycin-resistant growth around the disc. The glycopeptides teicoplanin, ristocetin and vancomycin (and the peptide bacitracin; data not shown) were particularly potent inducers. In contrast, 'negative control' antibiotics that target the ribosome (e.g. thiostrepton, streptomycin) or DNA gyrase (novobiocin) do not induce a halo.

Table 1 summarises the results of the assay for the antibiotics used in Figure 2 and for a further 11 antibiotics tested ( ✔ = induced a halo of kanamycin resistant growth, indicating ability to induce the *sigEp-neo* fusion; X= did not induce a halo of kanamycin resistant growth). A wide range of penicillins (e.g. penicillin G, oxacillin, ampicillin, ticarcillin), cephalosporins (e.g. cefaclor, cefadroxil, cephradine, cephalexin), glycopeptides (e.g. teicoplanin, ristocetin, vancomycin) and peptides (bacitracin) were found to induce the *sigE* promoter (Table 1). Thus it is clear that this *sigE* promoter bioassay detects structurally unrelated antibiotics with varied targets in the cell envelope, allowing the system to provide a broad-range, generic screen for cell envelope-specific antibiotics.

Although it is still unknown precisely which physical characteristic of the cell envelope the CseB/CseC signal transduction system responds too, these results clearly show the utility of the system as a screen. For instance, an initial assessment of sensitivity demonstrated that 75 ng of ristocetin gives a positive reaction in the bioassay.

It should be noted that many of the antibiotics acted as inducers without showing any signs of toxicity (e.g. vancomycin, Figure 2). With such antibiotics, the induced kanamycin-resistant halo grows to the edge of the antibiotic disc. The cell wall degradative enzyme lysozyme also acted as an inducer (data not shown), demonstrating that the *sigE* promoter bioassay can also be used to detect lytic enzymes that target the cell envelope.

| Penicillins | | Cephalosporins and other β-lactams | | Glycopeptides | | Peptides | | 'Negative Control' antibiotics that do not target the cell envelope | |
|---|---|---|---|---|---|---|---|---|---|
| Penicillin G | ✔ | Cefaclor | ✔ | Teicoplanin | ✔ | Bacitracin | ✔ | Novobiocin (target – DNA gyrase) | ✖ |
| Penicillin V | ✔ | Cefadroxil | ✔ | Ristocetin | ✔ | | | Thiostrepton (target – the ribosome) | ✖ |
| Amoxycillin | ✔ | Cephapirin | ✔ | Vancomycin | ✔ | | | Streptomycin (target – the ribosome) | ✖ |
| Ampicillin | ✔ | Cepharadine | ✔ | | | | | | |
| Mezlocillin | ✔ | Cefatrizine propylene glycol | ✔ | | | | | | |
| Piperacillin | ✔ | Cephazolin | ✔ | | | | | | |
| Ticarcillin | ✔ | Cefepime | ✔ | | | | | | |
| | | Cephalosporin | ✔ | | | | | | |
| | | Cephalexin | ✔ | | | | | | |

**Table 1.** 20 antibiotics known to target the cell envelope and three 'negative control' antibiotics that do not target the cell envelope were tested for their ability to induce the *sigEp-neo* fusion in the bioassay (✔ = induced a halo of kanamycin-resistant growth; ✖ = did not induce a halo of kanamycin-resistant growth). Nine examples of the primary data are shown in Fig. 2. The glycopeptides, teicoplanin, ristocetin and vancomycin, and the peptide bacitracin were particularly potent inducers.

Example 3 - use of reporter strain in bioassay

In order to perform the assay of the invention,spores of the host microorganism , for example, M600 carrying pIJ6880, are spread on MMT medium carrying a lethal concentration of kanamycin (100 µg ml$^{-1}$ )at concentration of approximately 5 x 10$^6$ / 12cm$^2$ plate. Test compound is applied on paper discs to a number of freshly spread plates in parallel using a different concentration of the test compound in each plate. A halo of kanamycin-resistant growth indicates that the test compound represents a cell envelope-specific antibiotic.

References

Aoki *et al.* (1976) Nocardicin A, a new monocyclic β-lactam antibiotic. I. Discovery, isolation and characterisation. *J. Antibiotics.* 29: 492-500.
Chakraburtty, R. and Bibb, M.J. (1997) The ppGpp synthetase gene (*relA*) of *Streptomyces coelicolor* A3(2) plays a conditional role in antibiotic production and morphological differentiation. *J Bacteriol* 179: 5854-5861.
Hopwood, D.A., Bibb, M..J., Chater, K.F., Kieser, T., Bruton, C.J., Kieser, H.M.., Lydiate, D.J., Smith, C.P., Ward, J.M., and Schrempf, H. (1985) Genetic Manipulation of *Streptomyces:* A Laboratory Manual. Norwich: The John Innes Foundation.
Imada, A., Kitano, K., Kintana, K., Muroi, M., and Asai, M. (1981) Sulfazecin and isosulfazecin, novel β-lactam antibiotics of bacterial origin. *Nature* 289: 590-591.
Janssen, G.R., and Bibb, M.J. (1993) Derivatives of pUC18 that have *Bgl*II sites flanking a multiple cloning site and that retain ability to identify recombinant clones by visual screening of *Escherichia coli* colonies. *Gene* 124: 133-134.
Katz, E., Thompson, C.J., and Hopwood, D.A. (1983) Cloning and expression of the tyrosinase gene from *Streptomyces antibioticus* in *Streptomyces lividnas. J Gen Microbiol* 129: 2703-2714.
Lonetto, M.A., Brown, K.L., Rudd, K.E., and Buttner, M.J. (1994) Analysis of the *Streptomyces coelicolor sigE* gene reveals the existence of a subfamily of eubacterial RNA polymerase σ factors involved in the regulation of extracytoplasmic functions. *Proc Natl Acad Sci USA* 91: 7573-7577.
Murakami, T., Holt, T.G., and Thompson, C.J. (1989) Thiostrepton-induced gene expression in *Streptomyces lividans. J Bacteriol* 171: 1459-1466.
Paget, M.S.B., Chamberlin, L. Atrih, A., Foster, S.J. and Buttner, M.J. (1999a) Evidence that the ECF sigma factor, σ$^E$, is required for normal cell wall structure in *Streptomyces coelicolor* A3(2). *J Bacteriol* 181: 204-211.
Paget, M.S.B., Leibovitz, E., and Buttner, M.J. (1999b) A putative two-component signal transduction system regulates σ$^E$, a sigma factor required for normal cell wall integrity in *Streptomyces coelicolor* A3(2). *Mol. Microbiol.* 33: 97-107.
Salah-Bey, K., Blanc, V., and Thompson, C.J. (1995) Stress-activated expression of a *Streptomyces pristinaespiralis* multidrug resistance gene (ptr) in various *Streptomyces* spp. and *Escherichia coli. Mol. Microbiol.* 17: 1001-1012.
Sykes, R.B *et al.* (1981) Monocyclic β-lactam antibiotics produced by bacteria. *Nature* 291: 489-491.
Sugiura, A., Hirokawa, K., Nakashima, K., and Mizuno, T. (1994) Signal-sensing mechanisms of the putative osmosensor KdpD in *Escherichia coli. Mol Microbiol* 14: 929-938.
Ulijasz, A.T., Grenader, A., and Weisblum, B. (1996) A vancomycin-inducible *lacZ* reporter system in *Bacillus subtilis:* induction by antibiotics that inhibit cell wall synthesis and by lysozyme. *J Bacteriol* 178: 6305-6309.
Walderhaug, M.O., Polarek, J.W., Voelkner, P., Daniel, J.M., Hesse, J.E., Altendorf, K., and Epstein, W. (1992) KdpD and KdpE, proteins that control expression of the *kdpABC* operon, are members of the two-component sensor-effector class of regulators. *J Bacteriol* 174: 2152-2159.
Ward, J.M., Janssen, G.R., Kieser, T., Bibb, M.J., Buttner, M.J. and Bibb, M.J. (1986) Construction and characterisation of a series of multi-copy promoter-probe plasmid vectors for *Streptomyces* using the aminoglycoside phosphotransferase gene from Tn5 as indicator. *Mol. Gen. Genet.* 203: 468-478.

**Claims**

**1.** A method of detecting in a sample the activity of an antibiotic which affects cell integrity, which method comprises the steps of:

   (a) providing a transformed microorganism which comprises a nucleic acid encoding a promoter operably linked to a heterologous reporter gene capable of causing a detectable signal,
   (b) contacting the sample with the transformed microorganism,

(c) observing said microorganism for said detectable signal, wherein the promoter is regulated by a two-component signal transduction system, which signal components are (i) a receptor responsive to changes in the cell envelope or membrane of the microorganism (ii) a trans-acting factor which is activated in response to stimulation by the receptor, and which regulates the promoter.

**characterised in that** the signal components are derived from CseB and CseC which regulate a *sigE* promoter.

2. A method as claimed in claim 1 wherein the reporter gene encodes a reporter protein which provides the microorganism with resistance to an antibiotic.

3. A method as claimed in claim 1 or claim 2 wherein the reporter gene encodes a reporter protein which can be detected photometrically.

4. A method as claimed in any one of the preceding claims wherein the promoter encoded by the nucleic acid is native to the microorganism or the species of the microorganism.

5. A method as claimed in any one of the preceding claims wherein the nucleic acid is in the form of an extrachromosomal vector.

6. A method as claimed in any one of the preceding claims wherein the some or all of the genes encoding the two component system are present at enhanced copy number in the transformed microorganism relative to the corresponding untransformed microorganism.

7. A method as claimed in any one of the preceding claims wherein some or all of the genes encoding the two component system are operably linked to a strong promoter such as to enhance their concentration in the microorganism.

8. A method as claimed in any one of the preceding claims wherein the microorganism is selected such as to have an enhanced sensitivity to cell wall-specific antibiotics.

9. A method as claimed in any one of the preceding claims wherein the microorganism is a bacterium.

10. A method as claimed in claim 9 wherein the bacterium is selected from the following species: *Streptomyces;* Mycobacteria.

11. A method as claimed in any one of the preceding claims wherein the sample is selected from: a culture supernatant; a soil isolate; the product of combinatorial chemical synthesis; the product of combinatorial biosynthesis.

12. A method as claimed in any one of the preceding claims wherein the activity is qualitatively correlated with the presence or absence of an antibiotic.

13. A method as claimed in any one of the preceding claims wherein the activity of the sample is further screened for antibiosis of a target organism.

14. A process of producing a transformed microorganism for use in any one of the preceding claims, which process comprises transforming a microorganism with a vector comprising (a) a promoter operably linked to (b) a heterologous reporter gene capable of causing a detectable signal, wherein the promoter is regulated by a two-component signal transduction system, which signal components are (i) a receptor responsive to changes in the cell envelope or membrane of the microorganism (ii) a trans-acting factor which is activated in response to stimulation by the receptor, and which regulates the promoter,
**characterised in that** the signal components are derived from CseB and CseC which regulate a *sigE* promoter.

15. A process as claimed in claim 14 wherein the copy number of the genes encoding the two component system is enhanced in the transformed microorganism relative to the corresponding untransformed microorganism.

16. A transformed microorganism for use in a method of any one of claims 1 to 13, which microorganism is transformed with a vector comprising (a) a promoter operably linked to (b) a heterologous reporter gene capable of causing a detectable signal, wherein the promoter is regulated by a two-component signal transduction system, which signal components are (i) a receptor responsive to changes in the cell envelope or membrane of the microorganism (ii) a trans-acting factor which is activated in response to stimulation by the receptor, and which regulates the promoter, **characterised in that** the signal components are derived from CseB and CseC which regulate a sigE promoter.

17. A transformed microorganism as claimed in claim 16 wherein the copy number of the genes encoding the two component system is enhanced in the transformed microorganism relative to the corresponding untransformed microorganism.

18. A kit for performing a method according to any one of claims 1 to 13, which kit comprises:

    (a) a preparation of the microorganism of claim 16 or claim 17, plus
    (b) further means for carrying out the contact or observation steps.

19. A kit as claimed in claim 18 comprising means for detecting the signal produced from the reporter gene.

20. A kit as claimed in claim 19 wherein the means are a photosensitive device.

21. A kit as claimed in claim 19 or claim 20 which is a parallel processing system in which detection of multiple activities is assessed using multiple cultures of transformed microorganisms.

**Patentansprüche**

1. Verfahren zum Detektieren der Aktivität eines die Zellintegrität beeinflussenden Antibiotikums in einer Probe, wobei das Verfahren folgende Schritte umfasst:

    (a) Bereitstellung eines transformierten Mikroorganismus, der eine für einen operabel an ein heterologes Reportergen, das zur Erzeugung eines detektierbaren Signals fähig ist, gebundenen Promotor kodierende Nucleinsäure umfasst,
    (b) Kontaktierung der Probe mit dem transformierten Mikroorganismus,
    (c) Beobachtung des Mikroorganismus hinsichtlich des detektierbaren Signals, worin der Promotor mittels eines Zwei-Komponenten-Signalübertragungssystems reguliert wird, wobei die Signalkomponenten (i) ein auf Veränderungen der Zellhülle oder Zellmembran des Mikroorganismus reagierender Rezeptor und (ii) ein Transkriptionsfaktor, der als Reaktion auf die Stimulation durch den Rezeptor aktiviert wird und der den Promotor reguliert, sind,

    **dadurch gekennzeichnet, dass** die Signalkomponenten von CseB und CseC abgeleitet sind, die einen sigE-Promotor regulieren.

2. Verfahren nach Anspruch 1, worin das Reportergen für ein Reporterprotein kodiert, welches dem Mikroorganismus mit Resistenz gegenüber einem Antibiotikum verleiht.

3. Verfahren nach Anspruch 1 oder 2, worin das Reportergen für ein Reporterprotein kodiert, das photometrisch detektiert werden kann.

4. Verfahren nach einem der vorangegangenen Ansprüche, worin der durch die Nucleinsäure kodierte Promotor ein nativer des Mikroorganismus oder der Spezies des Mikroorganismus ist.

5. Verfahren nach einem der vorangegangenen Ansprüche, worin die Nucleinsäure in Form eines extrachromosomalen Vektors vorliegt.

6. Verfahren nach einem der vorangegangenen Ansprüche, worin einige oder alle der für das Zwei-Komponenten-System kodierenden Gene im transformierten Mikroorganismus im Vergleich zu dem entsprechenden nichttransformierten Mikroorganismus in erhöhter Kopienzahl vorhanden sind.

**7.** Verfahren nach einem der vorangegangenen Ansprüche, worin einige oder alle der für das Zwei-Komponenten-System kodierenden Gene operabel an einen starken Promotor gebunden sind, um ihre Konzentration im Mikroorganismus zu erhöhen.

**8.** Verfahren nach einem der vorangegangenen Ansprüche, worin der Mikroorganismus so gewählt wird, dass er erhöhte Empfindlichkeit gegenüber zellwandspezifischen Antibiotika aufweist.

**9.** Verfahren nach einem der vorangegangenen Ansprüche, worin der Mikroorganismus ein Bakterium ist.

**10.** Verfahren nach Anspruch 9, worin das Bakterium aus folgenden Bakterienspezies ausgewählt ist: Streptomyces, Mycobakterien.

**11.** Verfahren nach einem der vorangegangenen Ansprüche, worin die Probe aus Folgendem ausgewählt ist: einem Kulturüberstand; einem Bodenisolat; dem Produkt von kombinatorisch-chemischer Synthese; dem Produkt von kombinatorischer Biosynthese.

**12.** Verfahren nach einem der vorangegangenen Ansprüche, worin die Aktivität qualitativ mit der Gegenwart oder Abwesenheit eines Antibiotikums korreliert wird.

**13.** Verfahren nach einem der vorangegangenen Ansprüche, worin die Aktivität der Probe weiters hinsichtlich Antibiose eines Zielorganismus gescreent wird.

**14.** Verfahren zur Herstellung eines transformierten Mikroorganismus zur Verwendung in einem der vorangegangen Ansprüche, wobei das Verfahren das Transformieren eines Mikroorganismus mit einem Vektor umfasst, umfassend (a) einen Promotor, der operabel an (b) ein heterologes, zur Erzeugung eines detektierbaren Signals fähiges Reportergen gebunden ist, worin der Promotor mittels eines Zwei-Komponenten-Signalübertragungssystems reguliert wird, wobei die Signalkomponenten (i) ein auf Veränderungen der Zellhülle oder Zellmembran des Mikroorganismus reagierender Rezeptor und (ii) ein Transkriptionsfaktor, der als Reaktion auf Stimulation durch den Rezeptor aktiviert wird und der den Promotor reguliert, sind,
**dadurch gekennzeichnet, dass** die Signalkomponenten von CseB und CseC abgeleitet sind, die einen sigE-Promotor regulieren.

**15.** Verfahren nach Anspruch 14, worin die Kopienzahl der für das Zwei-Komponenten-System kodierenden Gene im transformierten Mikroorganismus im Vergleich zu dem entsprechenden nichttransformierten Mikroorganismus erhöht ist.

**16.** Transformierter Mikroorganismus zur Verwendung in einem Verfahren nach einem der Ansprüche 1 bis 13, worin der Mikroorganismus mit einem Vektor, umfassend (a) einen Promotor, der operabel an (b) ein heterologes, zur Erzeugung eines detektierbaren Signals fähiges Reportergen gebunden ist, transformiert ist, worin der Promotor mittels eines Zwei-Komponenten-Signalübertragungssystems reguliert wird, wobei die Signalkomponenten (i) ein auf Veränderungen der Zellhülle oder Zellmembran des Mikroorganismus reagierender Rezeptor und (ii) ein Transkriptionsfaktor, der als Reaktion auf die Stimulation durch den Rezeptor aktiviert wird und der den Promotor reguliert, sind,
**dadurch gekennzeichnet, dass** die Signalkomponenten von CseB und CseC abgeleitet sind, die einen sigE-Promotor regulieren.

**17.** Transformierter Mikroorganismus nach Anspruch 16, worin die Kopienzahl der für das Zwei-Komponenten-System kodierenden Gene im transformierten Mikroorganismus im Vergleich zu dem entsprechenden nichttransformierten Mikroorganismus erhöht ist.

**18.** Set zur Durchführung eines Verfahrens nach einem der Ansprüche 1 bis 13, wobei das Set Folgendes umfasst:

(a) die Herstellung eines Mikroorganismus nach Anspruch 16 oder Anspruch 17 sowie
(b) weitere Mittel zur Durchführung der Schritte des Kontaktierens oder Beobachtens.

**19.** Set nach Anspruch 18, umfassend Mittel zum Detektieren des vom Reportergen erzeugten Signals.

**20.** Set nach Anspruch 19, worin das Mittel eine lichtempfindliche Vorrichtung ist.

**21.** Set nach Anspruch 19 oder Anspruch 20, das ein Parallelverarbeitungssystem ist, in dem die Detektion von mehreren Aktivitäten unter Verwendung mehrerer Kulturen des transformierten Mikroorganismus bewertet wird.

**Revendications**

**1.** Méthode de détection, dans un échantillon, de l'activité d'un antibiotique qui affecte l'intégrité des cellules, laquelle méthode comprend les étapes de:

(a) prévoir un microorganisme transformé qui comprend un acide nucléique codant pour un promoteur opérativement enchaîné à un gène reporteur hétérologue capable de produire un signal détectable,

(b) mettre ledit échantillon en contact avec le microorganisme transformé,

(c) observer ledit microorganisme pour ledit signal détectable, où le promoteur est régulé par un système de transduction de signaux à deux composants, lesquels composants de signaux sont (i) un récepteur répondant à des changements dans l'enveloppe de la cellule ou la membrane du microorganisme (ii) un facteur à transaction qui est activé en réponse à une stimulation par le récepteur, et qui régule le promoteur

**caractérisée en ce que** lesdits composants du signal sont dérivés de CseB et CseC qui régulent un promoteur *sigE.*

**2.** Méthode selon la revendication 1, où le gène .reporteur code pour une protéine reporteur qui donne au microorganisme une résistance à un antibiotique.

**3.** Méthode selon la revendication 1 ou la revendication 2, où le gène reporteur code pour une protéine reporteur qui peut être détectée photométriquement.

**4.** Méthode selon l'une quelconque des revendications précédentes, où le promoteur codé par l'acide nucléique est natif au microorganisme ou à l'espèce du microorganisme.

**5.** Méthode selon l'une quelconque des revendications précédentes, où l'acide nucléique est sous la forme d'un vecteur extrachromosomique.

**6.** Méthode selon l'une quelconque des revendications précédentes, où une partie ou la totalité des gènes codant pour le système à deux composants sont présents à un nombre amélioré de copies dans le microorganisme transformé relativement au microorganisme non transformé correspondant.

**7.** Méthode selon l'une quelconque des revendications précédentes, où une partie ou la totalité des gènes codant pour le système à deux composants sont opérativement enchaînés à un fort promoteur de façon à améliorer leur concentration dans le microorganisme.

**8.** Méthode selon l'une quelconque des revendications précédentes, où le microorganisme est sélectionné de façon à avoir une sensibilité améliorée aux antibiotiques spécifiques de la paroi des cellules.

**9.** Méthode selon l'une quelconque des revendications précédentes, où le microorganisme est une bactérie.

**10.** Méthode selon la revendication 9, où la bactérie est sélectionné parmi les espèces suivantes: *Streptomyces;* Mycobactéries.

**11.** Méthode selon l'une quelconque des revendications précédentes, où l'échantillon est sélectionné parmi: un produit surnageant d'une culture; un isolat de sol; le produit d'une synthèse chimique en combinaison; le produit d'une biosynthèse en combinaison.

**12.** Méthode selon l'une quelconque des revendications précédentes, où l'activité est quantitativement mise en corrélation avec la présence ou l'absence d'un antibiotique.

**13.** Méthode selon l'une quelconque des revendications précédentes, où l'activité de l'échantillon est de plus criblée pour une antibiose d'un organisme cible.

14. Procédé de production d'un microorganisme transformé à utiliser dans l'une quelconque des revendications précédentes, lequel procédé consiste à transformer un microorganisme avec un vecteur comprenant

(a) un promoteur opérativement enchaîné à (b) un gène reporteur hétérologue capable de produire un signal détectable, où le promoteur est régulé par un système de transduction de signaux à deux composants, lesquels composants de signaux sont (i) un récepteur répondant à des changements de l'enveloppe de la cellule ou de la membrane du microorganisme (ii) un facteur de trans -action qui est activé en réponse à une stimulation par le récepteur et qui régule le promoteur,

caractérisé en que les composants de signal sont dérivés de CseB et CseC qui régulent un promoteur *sig*E.

15. Procédé selon la revendication 14, où le nombre de copies des gènes codant pour le système à deux composants est amélioré dans le microorganisme transformé relativement au microorganisme non transformé correspondant.

16. Microorganisme transformé à utiliser dans une méthode de l'une quelconque des revendications 1 à 13, lequel microorganisme est transformé par un vecteur comprenant (a) un promoteur opérativement enchaîné à (b) un gène reporteur hétérologue capable de produire un signal détectable, où le promoteur est régulé par le système de transduction de signaux à deux composants, lesquels composants de signaux sont un récepteur répondant à des changements de l'enveloppe de cellule ou de la membrane du microorganisme (ii) un facteur de trans -action qui est activé en réponse à la stimulation par le récepteur, et qui régule le promoteur,
**caractérisé en ce que** les composants de signal sont dérivés de CseB et CseC qui régulent un promoteur *sig*E.

17. Microorganisme transformé selon la revendication 16, où le nombre de copies des gènes codant pour le système à deux composants est amélioré dans le microorganisme transformé relativement au microorganisme non transformé correspondant.

18. Kit pour accomplir une méthode selon l'une quelconque des revendications 1 à 13, lequel kit comprend:

(a) une préparation du microorganisme de la revendication 16 ou de la revendication 17, plus
(b) un autre moyen pour effectuer les étapes de contact ou d'observation.

19. Kit selon la revendication 18 comprenant des moyens pour détecter le signal produit à partir du gène reporteur.

20. Kit selon la revendication 19, où les moyens sont un dispositifs photosensible.

21. Kit selon la revendication 19 ou la revendication 20, qui est un système de traitement en parallèle dans lequel la détection des activités multiples, est évaluée en utilisant des cultures multiples de microorganismes transformés.

# Fig. 1. Model for the regulation of the *sigE promoter in response to signals from the cell envelope*

EP 1 287 158 B1

Penicillin G   Amoxycillin   Ticarcillin

Teicoplanin   Ristocetin   Vancomycin

Thiostrepton   Streptomycin   Novobiocin

FIG. 2

EP 1 287 158 B1